(19) Europäisches Patentamt
European Patent Office
Office européen des brevets

(11) Publication number: **0 236 684 B1**

(12) # EUROPEAN PATENT SPECIFICATION

(45) Date of publication of patent specification: **20.05.92**

(51) Int. Cl.5: **A61K 31/55**

(21) Application number: **87100461.0**

(22) Date of filing: **15.01.87**

The file contains technical information submitted after the application was filed and not included in this specification

(54) **Galanthamine or analogues thereof for treating Alzheimer's disease.**

(30) Priority: **15.01.86 US 819141**

(43) Date of publication of application:
**16.09.87 Bulletin 87/38**

(45) Publication of the grant of the patent:
**20.05.92 Bulletin 92/21**

(84) Designated Contracting States:
**AT BE CH DE ES FR GB GR IT LI LU NL SE**

(56) References cited:

**PSYCHIAT. NEUROL. MED. PSYCHOL., vol. 23, December 12, 1971, pages 712-718; Leipzig,DD W.GÖPEL et al.: "Erfahrungen mit Nivalin in der neurologischen Therapie"**

**CHEMICAL ABSTRACTS, vol. 81, no. 13, September 30, 1974, page 23. ref. no. 72615z; Columbus, Ohio, US; W.A.KRAUZ: "Correlations between the ventral and dorsal hippocampus in the improvement of deterioration of short-term memory"**

(73) Proprietor: **SYNAPTECH, INC.**
**17 Seacrest Drive**
**Huntington, New York 11743(US)**

(72) Inventor: **Davis, Bonnie M.**
**17 Seacrest Drive**
**Huntington, New York 11743(US)**

(74) Representative: **Baillie, Iain Cameron et al**
**c/o Ladas & Parry, Altheimer Eck 2**
**W-8000 München 2(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.08/2.19/2.0)

R.BERKOW: "THE MERCK MANUAL OF DIAGNOSIS AND THERAPY", 14th Ed., 1982; Merck Sharp & Dohme Research Laboratories, US pages 1305-1309: "Dementia"

THE LANCET, January 6, 1979, page 42 C.M. SMITH et al.: "Physostigmine in Alzheimer's disease"

Katzman New England Journal of Medicine April 10, 1986

Kendall et al, J Clin & Hosp Pharmac(1985) 10: 327-336

NOUV. PRESSE MED., vol. 7, no. 45, 1978, page 4152 D.A. COZANITIS: "L' hydrobromide de galanthamine: un substitut de sulfate d'éserine (physostigmine) pour le traitement des effets cérébraux des substances anticholinergiques"

Hyppocampus, Encyclopedia of Neuroscience - G Adelman Ed, Birkhauser, Boston 1987

Stahl et al in Cognitive Neurochemistry, page 83

Science, vol. 225, pages 1168-1170

The Hippocampus, Jarrard, Vol IV Isaacon & Pribram eds (1986) pp. 93-126

The Pharmacologic Basis of Therapeutics, Weiner, AG Goodman et al, eds, Macmillan, New York, 1985, p. 148

Byull Eksp Biol Med 83(2): 185-187, Feb. 1977

J Am Geriat Soc (1977) 25:289

American Journal of Psychiatry, 138:5, 1981, p. 59, Reisberg B et al5

Psychopharmacology 52: 251-255, 1977, Crook T et al

The Pharmacological Basis of Therapeutics, p. 583-585, Franz DN

J. Med. Chem 29 (7): 1125-1130, 1986, Hershenson et al

J Pharm Pharmac (1977) 29:110

Pharmacol Biochem Behav (1976) 5: (Suppl.)41

J Clin Hosp Pharmac (1985) 10:327

Neurology (1985) 35:1348

Arzneim Forsch (1976) 26:1947

Acta Physiol Pharmacol Bulg (1976) 2:49

Brain Research (1975) 84 329-335

J Am Geriat Soc (1977) 25:289

Sachar EJ ed. Hormones Behavior and Psychopathology; New York, Raven Press, 1976, p. 1

Neurobiology of Aging (1985) 6:95

J Pharmacol (1985) 16 Suppl 3:39-49

N Engl J Med (1990) 323 (7) 445-8

Pharmacol Biochem Behav (1978) 8(3): 215-8

Am J Psychiat (1987) 144(4): 468-71

Exp Neurol (1983) 81:330-339

Arch Neurol (1990) 47(10):1126-30

Psychopharmacology (1984) 84 (4) 496-502

Arch Neurol (1985) 42(8): 744-8

Am J Psychiat 138(5) p 594-596 (1981)

Sunderland et al, Alzheimer's Disease Current Research in Diagnosis; Taylor & Francis, New York 1990, Robert Becker and Ezio Giacobini eds.

Animal Models of Psychiatric Disorders 1:175-194, 1988

## Description

## General Field of the Invention

The present invention relates to a novel use of galanthamine or an analogue or a pharmaceutically acceptable acid addition salt thereof for preparing a medicament for the treatment of Alzheimer's disease or related dementias.

## Background Art

Galanthamine and acid addition salts thereof have, for many years, been known to have anticholinesterase properties. Cozanitis in Anaesthesia 29 163-8 (1974) describes the effect of galanthamine hydrobromide on plasma cortisol of patients receiving relaxant anaesthesia and Cozanitis et al in Acta Anesth. Scand. 24:1166-168 (1980) describe the effect of galanthamine on plasma ACTH values during anaesthesia. These studies showed an increase in both plasma cortisol and plasma ACTH when galanthamine was administered to patients together with atropine. Cozanitis also suggested that galanthamine had a larger period of action as a cholinesterase inhibitor than physostigmine.

Il'yuchenok et al (Chemical Abstracts 70 36296K) describe the appearance of O-rhythm on an electroencephalogram when galanthamine is administered intravenously to rabbits.

Increase in short-term memory in dogs by use of galanthamine is described by Krauz in Chemical Abstracts 81 72615Z. Although short-term memory loss is one feature of Alzheimer's disease, the disease is much more complicated than this. Many drugs have been found to have beneficial effects on short-term memory in normal subjects but have been ineffective in treating Alzheimer's disease.

The antagonistic effect of galanthamine to scopolamine-induced amnesia in rats is described by Chaplygina et al in Chemical Abstracts 86 115157Z, and in Zhurnal Vysshei Nervnoi Deiatelnosti imeni P. Pavlova (MOSKVA) 26:1091-3, 1976.

Goepel in Psychiat Neurol Med Psychol Vol. 23 (1971) compares prostigmine (neostigmine) and pyridostigmine with galanthamine and suggests that galanthamine may be useful to treat a variety of "neurological disorders". However, neither neostigmine nor pyridostigmine have proved to be useful in treating Alzheimer's disease.

A letter to The Lancet, January 6, 1979 by Smith and Swash indicates improvements in memory in Alzheimer's disease patients by use of physostigmine. Physostigmine has been tried extensively in clinical trials for treatment of Alzheimer's disease but has not achieved any generally recognized acceptance.

Alzheimer's disease, presenile dementia, causes much distress not only to those suffering from the disease, but also those who are close to them. The custodial care of advanced victims of the disease is a tremendous expense to society. At present, there is no effective means of improving the functional status of persons with the disease.

It is an object of the present invention to improve the cognitive function of patients with Alzheimer's disease.

A radioactively-labelled form of the molecule may also serve as a diagnostic test for Alzheimer's disease.

## Detailed Description of the invention

Galanthamine is generally regarded as having the structure:

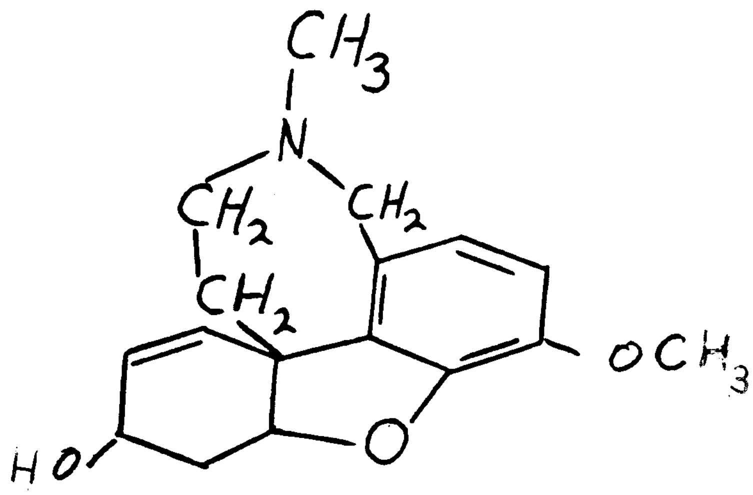

Compounds of a similar structure wherein the hydroxy is replaced by methoxy, ethoxy, lower alkanoyl oxy such as acetyloxy, the methoxy group is replaced by hydrogen, ethoxy, or lower alkanoyloxy such as acetyloxy and the methyl group substituted on the nitrogen atom is replaced by other straight or branch chain lower alkyl groups such as ethyl, cyclopropylmethyl or cyclobutylmethyl, allyl, lower alkyl phenyl or substituted lower alkyl phenyl wherein the substituents are fluoro, chloro, bromo, lower alkoxy, hydroxy, nitro, amino lower alkyl or acylamino of from 1 to 5 carbon atoms, or unsubstituted and halogen substituted benzoyl lower alkyl in which the substituents are on the phenyl ring, and compounds wherein hydrogen atoms in the "core" structure have been replaced by fluoro or chloro groups are likely to have similar properties to galanthamine. When used herein the term "galanthamine or its analogues" means galanthamine and galanthamine derivatives which one or more of the above-mentioned replacements of hydrogen, methoxy or methyl groups have been effected. Conversion of galanthamine to its analogues can be accomplished by steps well known to those skilled in the art, for example, converting the hydroxyl groups to an alkyl group by use of a dehydrating catalyst in a reaction with an alcohol or by a Williamson reaction, and by esterifying a hydroxy group to form an alkanoyloxy group, for example, by use of acetic anhydride. Alternatively, many of these compounds may be synthesized by normal chemical techniques.

Galanthamine or an analogue can be administered in any convenient chemical or physical form. For example, it may be administered as its hydrobromide, hydrochloride, methylsulfate or methiodide.

Galanthamine or an analogue or its pharmaceutically-acceptable acid addition salts may be administered to a patient suffering from Alzheimer's disease orally or by subcutaneous or intravenous, injection, or intracerebroventricularly by means of an implanted reservoir. It may be necessary to begin at lower doses than are ultimately effective.

Galanthamine and its acid addition salts form crystals. They are in general only sparingly soluble in water at room temperature and so injectible compositions are normally in the form of an aqueous suspension. If necessary, pharmaceutically-acceptable suspension aids may be employed. Typically, such a suspension will be employed at a concentration of 1-50 mg/ml more commonly 5-40 mg/ml, for example, 5-30 mg/ml or 10-40 mg/ml, typically 20-30 mg/ml of galanthamine. Typical dosage rates when administering galanthamine or an analogue will depend upon the exact nature of the compound used and the condition of the patient. Thus for treatment with galanthamine itself or a salt thereof typical dosage rates by injection are in the range 5-1,000 mg per day depending upon the patient. In some cases even lower dosages, as low as 0.5 or 1 mg per day may be helpful. For example, divided doses in the range 0.5-5 mg/kg body weight per day may prove useful. Typically, one might administer a dosage of 50-300 mg per day to a patient of a body weight of 40-100 kg, although in appropriate cases such dosages may prove useful for patients having a body weight outside this range. In other cases, dosages as low as 10 mg and as high as 500 mg may be appropriate for persons in this body weight range.

Galanthamine or an analogue or its pharmaceutically-acceptable acid addition salts may also be administered orally, for example, as an aqueous suspension or a solution in aqueous ethanol or as a solid such as a tablet or capsule. Suspensions or solutions for oral administration are typically of about the same concentration as those used for injections. However, it may be desirable when administering the drug orally to use a higher dosage rate than when administering it by injection. For example, dosages up to 2000 mg per day may be used, such as dosages in the range 100-600 mg per day. In preparing such tablets or capsules, standard table or capsule-making techniques may be employed. The dosage rate of galanthamine or its pharmaceutically-acceptable salt will normally be in the same range as for oral administration of a liquid. If desired, a pharmaceutically-acceptable carrier such as starch or lactose may be used in preparing galanthamine tablets. Capsules may be prepared using soft gelatine as the encapsulating agent. If desired, such capsules may be in the form of sustained release capsules wherein the main capsule contains microcapsules of galanthamine which release the contents over a period of several hours thereby maintaining a constant level of galanthamine in the patient's blood stream.

The following test provides a good animal model of Alzheimer's disease in humans: A selective lesion is placed in a subcortical nucleus (nucleus basalis of Meynert) with a resultant cortical cholinergic deficiency, similar in magnitude to that seen in early to moderate stage of Alzheimer's disease. Numerous behavioral deficits, including the inability to learn and retain new information, characterizes this lesion. Drugs that can normalize these abnormalities would have a reasonable expectation of efficacy in Alzheimer's disease. Haroutunian, V, Kanof P, Davis, KL: Pharmacological alleviations of cholinergic-lesion-induced memory defects in rats. Life Sciences 37:945-952, 1985.

The following specific formulations may find use in treatment of Alzheimer's disease:

Tablets or capsules containing 5, 10 and 25 mg galanthamine hydrobromide to be taken four times a day, or a sustained-release preparation delivering an equivalent daily dose.

Parenteral solution containing 5 mg/ml.

Liquid formulation for oral administration available in 5 mg/5ml and 25 mg/5ml concentration.

There have been reports that galanthamine can cause cardiac arrythmias. In such cases, it may be desirable to administer galanthamine in conjunction with another drug such as propanthelinbromide to control such arrythmias.

## Claims

1. Use of galanthamine or an analogue or a pharmaceutically acceptable acid addition salt thereof for preparing a medicament for the treatment of Alzheimer's disease or related dementias.

2. A medical device for use in the treatment of Alzheimer's disease and related dementias capable of administering a galanthamine or a pharmaceutically acceptable salt thereof intracerebroventricularly from a reservoir at a dosage rate of 0.01 to 5.0 mg/kg/day.

## Revendications

1. Utilisation de la galantamine, d'un de ses analogues ou d'un de ses sels d'addition d'acide pharmaceutiquement acceptables pour la préparation d'un médicament destiné au traitement de la maladie d'Alzheimer ou de démences apparentées.

2. Dispositif médical pour utilisation dans le traitement de la maladie d'Alzheimer et des démences apparentées, ledit dispositif étant capable d'administer la galantamine ou l'un de ses sels pharmaceutiquement acceptables, intracerébroventriculairement à partir d'un réservoir à un taux de 0,01 à 5,0 mg/kg/jour.

## Patentansprüche

1. Verwendung von Galanthamin oder einer analogen Substanz oder eines pharmazeutisch verwendbaren Säureadditionssalzes des- bzw. derselben zum Herstellen eines Arzneimittels zur Behandlung der Alzheimerschen Krankheit oder damit verbundener Demenzen.

**2.** Medizinische Vorrichtung für die Verwendung bei der Behandlung der Alzheimerschen Krankheit oder damit verbundener Demenzen, wobei die Vorrichtung zur intracerebroventrikulären Applikation eines Galanthamins oder eines pharmazeutisch verwendbaren Salzes desselben aus einem Vorratsbehälter in einer Dosierung von 0,01 bis 5,0 mg/kg/Tag geeignet ist.